# EUROPEAN PATENT APPLICATION

(11) **EP 1 746 098 A1**
(43) Date of publication of application: **24.01.2007**
(21) Application number: 05015857.5
(22) Date of filing: 21.07.2005
(51) Int. Cl.: C07D 473/18, A61K 31/522, A61P 31/00

(54) **Valacyclovir polymorphs and a process for the preparation thereof**

(71) Applicant: SOLMAG S.p.A., 26837 Mulazzano LO (IT)
(72) Inventor: Pizzocaro, Francesco, 20024 Garbagnate (MI) (IT)
(74) Representative: Minoja, Fabrizio

(57) **Abstract**

A process for the preparation of valacyclovir hydrochloride in monohydrate form, as well as of several other hydrate and anhydrous forms, and novel hydrate polymorphic forms obtainable by said process.

## Description

The present invention provides a process for the preparation of valacyclovir hydrochloride in monohydrate form, as well as of several other hydrate and anhydrous forms.

The invention also relates to novel hydrate polymorphic forms obtainable by said process.

### Background of the invention

Valacyclovir or 1-valine, 2-[(2-amino-1,6-dihydro-6-oxo-9H-purin-9-yl)methoxy]ethyl ester is known to exhibit polymorphism. Several forms have been reported since late nineties. Valacyclovir is the L-valinate ester pro-drug of acyclovir, and it was firstly disclosed in EP 0308065. Acyclovir has high anti-viral activity and is widely used in the treatment or prophylaxis of viral infections in humans. No particular crystalline form was claimed in the original patent, but in the examples a Valacyclovir hydrochloride monohydrate form (theor: 4.75% water) was disclosed. The same example also teaches a crystallization process from ethanol/water providing a C,H,N analysis. Other examples describe some derivatives such as L-isoleucinate in the anhydrous forms.

US 6,107,302 discloses a Valacyclovir anhydrous form with water content up to about 3%, and obtained with a modified process vs. the previous one. X-ray diffraction patterns were also reported. The process included an acetone precipitation, drying at 60°C, suspension in ethanol overnight at 60°C, and then drying again at 60°C. The same patent, in addition to the previously disclosed monohydrate, discloses a dihydrate form (with a theoretical water content of about 9.8%). An additional anhydrous form is disclosed in WO 03/040145.

WO 03/022209 discloses several additional crystalline forms including the already known monohydrate. However, in addition to the previous ones, a new crystalline form, namely a sesquihydrate, containing 1.5 molecules of water per molecule of Valacyclovir hydrochloride, was disclosed with a water content of about 6.9%.

Additional forms were also disclosed in WO 04/106338. A monohydrate crystalline form prepared by precipitation from DMF by acetone addition was also disclosed in this patent.

Several forms of valacyclovir are therefore known, as well as several different solvates or hydrates, in particular a number of discrete hydrates. The monohydrate and a process for its preparation was firstly disclosed in EP 0308065. In general, however, each form, hydrate or solvate is obtained according to the prior art by a different, peculiar process.

It is well known that crystalline forms of a pharmaceutically useful compounds may affect the performance characteristic of a pharmaceutical product. See G. M. Wall, *Pharm. Manuf.* **3,** 33 (1986); J.K. Haleblian and W. McCrone, *J*. *Pharm. Sci.,* **58,** 911 (1969); and J. K. Haleblian, *J. Pharm. Sci.,* **64**, 1269 (1975). Another important solid state property of a pharmaceutical compound is its rate of dissolution in aqueous fluids, that can have therapeutic, formulation, and storage stability consequences.

EP 0308065, although disclosing the process for the preparation of monohydrate hydrochloride in ethanol/water, does not provide details concerning the experimental crystallization conditions used.

It has now surprisingly been found that small differences in said ethanol/water mixture may lead to different forms and/or solvates, i.e. anhydrous, monohydrate, sesquihdrate or dihydrate selectively. Thus, object of the present invention is a process for the preparation of each desired hydrate or even anhydrous form by just adjusting the % of water in ethanol during the crystallization. More importantly, it has also surprisingly been found that, independently of the water content in valacyclovir hydrochloride at the end of crystallization and drying, after equilibration, the final water content mainly depends on the way the crystallization is performed, and does not change in time. This means that the so obtained hydrates are also stable in time.

In particular, a further object of the present invention is a process for the selective preparation of valacyclovir in the monohydrate hydrochloride form, under a very well defined range of conditions, and in a highly repeatable, controlled manner. A further object of the invention is therefore also to provide a process that selectively affords valacyclovir as the monohydrate hydrochloride, that may be useful to prepare any desired pharmaceutical composition.

It is advantageous and desirable to adjust the reaction conditions to make the preparation of the desired hydrate form possible, using inexpensive non toxic solvents, namely water/ethanol mixtures. Furthermore, it is very important from the industrial point of view to define conditions allowing the scale-up preparation of the desired crystalline form.

### Summary of the invention

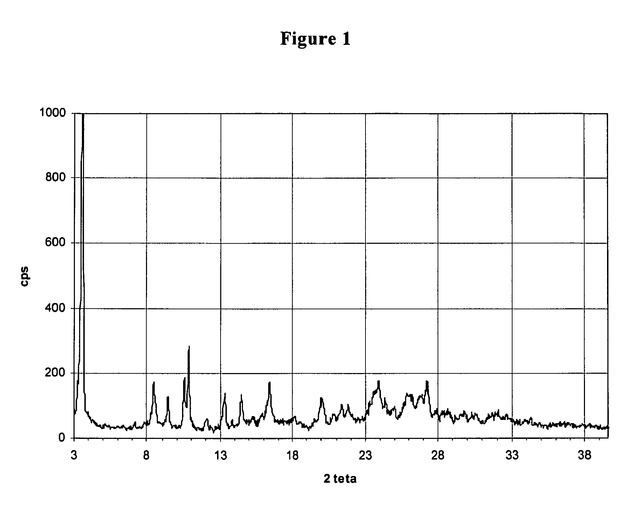

The present invention provides Valacyclovir hydrochloride monohydrate in a crystalline form, having a X-ray diffraction pattern, and typical IR spectra of FIG. 1-2, and a K.F. value comprised in the range 3.3-5.5%. In addition other known forms, both anhydrous and solvates, may optionally be obtained, according to the crystallization conditions.

In particular, anhydrous forms, with a K.F. value up to 3.0%, sesquihydrate form, K.F. comprised between 6-8%, and a dihydrate form, with a K.F. comprised between 8-10%, may also be optionally obtained. Other hydrates, with K.F. up to 15% may also optionally be obtained.

Another feature of the invention is to provide a process for the preparation of said Valacyclovir hydrochloride monohydrate in a stable crystalline form, which comprises dissolving crude Valacyclovir hydrochloride, obtained by a known process, in a solvent at a suitable temperature. Typical solvents include C₁-C₄ alcohols, in presence of amounts of water ranging from 2.5 to 80%, and suitable temperatures include temperatures ranging from 10°C to 100°C. A C₁-C₄ alcohol is added at the same temperature, and then the solution is maintained at such temperature for a time varying from 10 minutes to overnight. The resulting mixture is then cooled to a temperature ranging from -5°C to 45°C., then stirred at the above temperature for a time ranging from 1 hour to 2 days. The solid is filtered and washed with a solvent selected from C₁-C₄ alcohols, optionally in presence of amounts of water. The cake is then washed with a solvent, as before defined, subsequently dried at a suitable temperature under vacuum, optionally with controlled humidity and optionally in presence of an inert atmosphere, to yield Valacyclovir hydrochloride monohydrate. Suitable temperatures include temperatures ranging from room temperature to 90°C.

### Brief description of the drawings

FIG. 1-2 show a representative powder X-ray diffraction pattern, and IR spectra of Valacyclovir hydrochloride monohydrate crystalline form, respectively.

X-ray diffraction patterns were measured on an automated diffractometer θ/θ Ital Structures with CuKr radiation. IR spectra were recorded on a Spectrum One FT-IR Spectrometer Perkin-Elmer, by a diffuse reflectance method.

### Detailed description of the invention

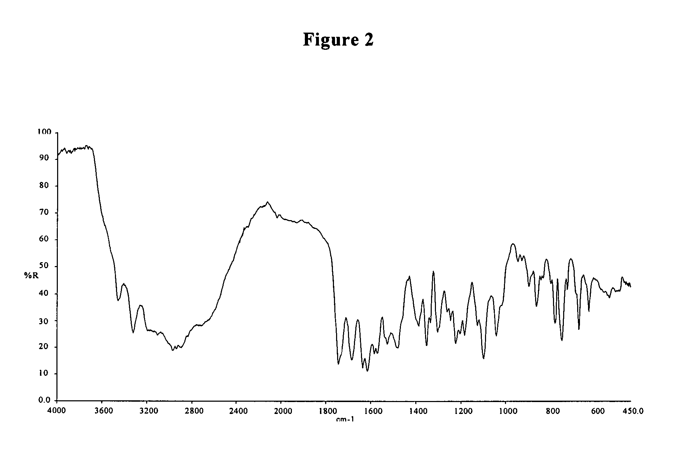

The present invention provides Valacyclovir hydrochloride monohydrate crystalline form having an X-ray diffraction pattern as reported FIG. 1, and an IR spectra as reported in FIG. 2. The crystalline form is also characterized by X-ray diffraction peaks (reflections) at about: 3.6, 8.5, 9.4, 10.8, 12.1, 13.3, 14.5, 16.4, 20.0, 21.4, 23.7, 25.9, 27.2, 28.5 ± 0.1 degrees two-theta.

Another feature of the invention is to provide a process for the preparation of the crystalline form of Valacyclovir hydrochloride monohydrate, which comprises the following steps:
i) Dissolving Valacyclovir hydrochloride obtained by a known process in a suitable solvent;
ii) Adding C₁-C₄ alcohols;
iii) Maintaining the solution at the same above said suitable temperature for a suitable time period;
iv) Cooling the resulting mixture;
v) Stirring the resulting suspension;
vi) Filtering the suspended crystalline solid;
vii) Washing the cake with a suitable solvent;
viii) Drying the wet cake under vacuum;
ix) Repeating the steps i) to viii) above, if needed; or
x) Optionally suspending crude Valacyclovir hydrochloride in the solvent of steps i) and ii), then performing the steps from v) to viii) as described above.

Step i) is carried out in solvents selected from C₁-C₄ alcohols, preferably C₁-C₃ alcohols, in presence of water in amounts ranging from 2.5 to 80%, preferably from 5 to 50%, at temperatures ranging from 10°C to 100°C, preferably from 45°C to 90°C.

Step ii) is carried out under the same conditions as step I).

Step iii) is carried out at the same temperatures as above, for a time ranging from 10 minutes to several hours, preferably from 30 minutes to 8 hours.

Step iv) is carried out at temperatures ranging from -5°C to 45°C, preferably from 10°C to 40°C.

Step v) is carried out at the same temperatures as in step iv), for a time ranging from 1 hour to 2 days, preferably from 5 hours to 1 day.

Step vii) is carried out in solvents selected from C₁-C₄ alcohols, preferably C₁-C₃ alcohols, in the presence of variable amounts of water.

Step viii) is carried out optionally in the presence of controlled % of humidity and optionally in presence of an inert atmosphere.

Step x) is carried out at temperatures ranging from room temperature to 90°C, preferably from 20°C to 70°C, most preferably temperatures range from 20°C to 60°C.

Most preferably the process is performed with the same hydro-alcoholic solutions from step ii) through step vii), namely in water-ethanol mixtures with a water to ethanol ratio ranging from 7.5% to 12.5%.

Most preferred temperatures for steps i) through step iii) range from 45°C to 85°C.

Most preferred temperatures for steps iv) and v) range from 10°C to 45°C.

In addition, during the crystallization the solution may optionally be seeded with valacyclovir hydrochloride monohydrate crystalline form, obtained as described above.

The resulting monohydrate has a K.F. value (water content) that may be comprised between 2% and 7% w/w, preferably between 3% and 6% w/w, most preferably between 3.5% and 5.5%.

According to the invention, other forms may optionally be obtained by suitably increasing or reducing the water content during the crystallization in steps i) to vii). In particular, anhydrous form may be obtained with low water contents, preferably below 7.5%, while sesquihydrate and dihydrate forms may be optionally obtained with higher water contents in the hydro-alcoholic crystallization solution, preferably ranging from 12.5% to 20%.

The resulting anhydrous form has a K.F. value (water content) that may be comprised between 0% and 3% w/w, as disclosed in US 6,107,302. The sesquihydrate form may have a water content, as determined by K.F., ranging from 6% to 8%. The dihydrate form may have a water content, as determined by K.F., that may range between 8% and 10% w/w.

The composition containing Valacyclovir hydrochloride monohydrate crystalline form may be in a form suitable for the oral administration, such as a tablet, capsule, suspension, or the topical administration, such as an ointment, cream or a lotion. These formulations may contain additional additives, sweetening and flavouring agent, coating and inert diluents, such as lactose and talc, binders and suspending agents, such as starch, hydroxyethylcellulose, hydroxypropyl cellulose, and the like. Any conventional technique may be used for the preparation of the pharmaceutical formulations according to the invention.

The invention is illustrated in further detail by the following Examples.

### Example 1

### Valacyclovir hydrochloride monohydrate

100 g of crude Valacyclovir hydrochloride, obtained by known processes, are suspended in a mixture of absolute ethanol (100 ml) and deionised water (100 ml). The resulting suspension is refluxed until complete dissolution. 800 ml of absolute ethanol are then added at the same temperature, and the solution is stirred for three hours at a temperature of about 70-75°C. The mixture is then cooled to room temperature and stirred overnight. The resulting suspension is filtered, and the cake is dried at 40-45°C under vacuum in a controlled humidity (saturated) atmosphere. The desiccated product is then equilibrated at room temperature. The title compound is obtained, with a K.F. of about 4.6%. X-ray diffraction pattern, and IR are as reported in FIG. 1-2.

### Example 2

### Valacyclovir hydrochloride monohydrate

200 g crude Valacyclovir hydrochloride hydrate, obtained by known processes, are suspended in absolute ethanol (2 1). The suspension is stirred for 12-14 hours at 20-25°C, then filtered. The cake is dried at 40-45°C under vacuum under controlled humidity (saturated) atmosphere. The desiccated product is then equilibrated at room temperature. The title compound is obtained, with a K.F. of about 4.6%. X-ray diffraction pattern, and IR are as reported in FIG. 1-2.

### Example 3

### Anhydrous Valacyclovir hydrochloride

30 g of crude Valacyclovir hydrochloride, obtained by known processes, are suspended in a mixture of absolute ethanol (30 ml) and deionised water (22.5 ml). The resulting suspension is heated until complete dissolution. 260 ml of absolute ethanol are then added at the same temperature. The mixture is cooled to room temperature and stirred overnight. The resulting suspension is filtered, and the cake is dried at 40°C under vacuum. The title compound is obtained, with a K.F. of about 0.6-0.8%.

### Example 4

### Valacyclovir hydrochloride sesquihydrate

30 g of crude Valacyclovir hydrochloride, obtained by known processes, are suspended in a mixture of absolute ethanol (30 ml) and deionised water (33 ml). The resulting suspension is heated until complete dissolution. 260 ml of absolute ethanol are then added at the same temperature. The mixture is cooled to room temperature and stirred overnight. The resulting suspension is filtered, and the cake is dried at 40°C under vacuum in a controlled humidity (saturated) atmosphere. The desiccated product is then equilibrated at room temperature. The title compound is obtained, with a K.F. of about 7%.

### Example 5

### Valacyclovir hydrochloride dihydrate

50 g of crude Valacyclovir hydrochloride, obtained by known processes, are suspended in a mixture of absolute ethanol (100 ml) and deionised water (100 ml). The resulting suspension is heated until complete dissolution. 900 ml of absolute ethanol are then added at the same temperature. The mixture is cooled to room temperature and stirred overnight. The resulting suspension is filtered, and the cake is dried at 40°C under vacuum. The desiccated product is then equilibrated at the air at room temperature. The title compound is obtained, with a K.F. of about 8.8%.

## Claims

1. Valacyclovir hydrochloride monohydrate **characterized by** a X-ray diffraction powder pattern shown in FIG. 1, and IR spectra shown in FIG. 2.

2. Valacyclovir hydrochloride monohydrate according to claim 1, **characterized by** a powder X-ray diffraction pattern having the characteristic X-ray diffraction peaks (reflections) at about: 3.6, 8.5, 9.4, 10.8, 12.1, 13.3, 14.5, 16.4, 20.0, 21.4, 23.7, 25.9, 27.2, 28.5 ± 0.1 degrees two-theta.

3. A process for the preparation of crystalline Valacyclovir hydrochloride monohydrate, which comprises the following steps:
i) Dissolving Valacyclovir hydrochloride obtained by a known process in a suitable solvent;
ii) Adding C₁-C₄ alcohols;
iii) Maintaining the solution at the same above said suitable temperature for a suitable time period;
iv) Cooling the resulting mixture;
v) Stirring the resulting suspension;
vi) Filtering the suspended crystalline solid;
vii) Washing the cake with a suitable solvent;
viii) Drying the wet cake under vacuum;
ix) Optionally repeating the steps i) to viii) above, if needed; or
x) Optionally suspending crude Valacyclovir hydrochloride in the solvent of steps i) and ii), then performing the steps from v) to viii) as described above.

4. A process according to claim 3 wherein the suitable solvents are ethanol/water mixtures.

5. A process according to claim 3 wherein temperatures range from 0°C to the solvent reflux temperature.

6. A process according to claim 3 wherein the mixture is seeded with valacyclovir crystalline form.

7. A process for the preparation of hydrated forms of Valacyclovir hydrochloride by crystallization from hydro-alcoholic solutions of Valacyclovir hydrochloride, **characterised in that** the amount of water in said hydro-alcoholic solution is adjusted from below 7.5%, whereby the anhydrous form is obtained, to 12.5%- 20%, whereby the sesquihydrate and dihydrate forms are obtained.

8. A pharmaceutical composition comprising said Valacyclovir hydrochloride crystalline forms and a pharmaceutically acceptable carrier.
